(19) **Europäisches Patentamt**
**European Patent Office**
**Office européen des brevets**

(11) Numéro de publication : **0 431 990 B1**

(12) **FASCICULE DE BREVET EUROPEEN**

(45) Date de publication du fascicule du brevet :
**15.12.93 Bulletin 93/50**

(51) Int. Cl.$^5$ : **C07C 15/02,** C07C 2/76,
C07C 2/66

(21) Numéro de dépôt : **90402989.9**

(22) Date de dépôt : **24.10.90**

(54) **Procédé de production d'hydrocarbures alkylaromatiques à partir de gaz naturel.**

(30) Priorité : **28.11.89 FR 8915767**

(43) Date de publication de la demande :
**12.06.91 Bulletin 91/24**

(45) Mention de la délivrance du brevet :
**15.12.93 Bulletin 93/50**

(84) Etats contractants désignés :
**BE CH DE ES FR GB IT LI NL SE**

(56) Documents cités :
**EP-A- 0 366 515**
**FR-A- 2 552 078**
**FR-A- 2 589 859**

(73) Titulaire : **INSTITUT FRANCAIS DU PETROLE**
**4, Avenue de Bois Préau**
**F-92502 Rueil-Malmaison (FR)**

(72) Inventeur : **Juguin, Bernard**
**46, avenue du Stade**
**F-92500 Rueil Malmaison (FR)**
Inventeur : **Collin, Jean-Claude**
**10, rue Agrippa d'Aubigné Marsinval**
**F-78240 Vernouillet (FR)**
Inventeur : **Larue, Joseph**
**97, Grande Rue**
**F-78240 Chambourcy (FR)**
Inventeur : **Busson, Christian**
**47 avenue Bergeron**
**F-69260 Charbonnière (FR)**

## Description

La présente invention concerne un procédé de production d'hydrocarbures alkylaromatiques à partir de gaz naturel.

Jusqu'à maintenant, les principaux procédés de production d'hydrocarbures alkylaromatiques étaient le réformage, le vapocraquage et l'alkylation. Plus récemment, un nouveau procédé d'aromatisation des paraffines légères a été évoqué.

Le procédé selon l'invention se compose de trois phases principales successives :

1) craquage thermique du gaz naturel renfermant du méthane (par exemple 60 à 99 % en volume) avec formation d'hydrogène et d'hydrocarbures $C_2^+$,

2) séparation des hydrocarbures $C_2^+$, obtenus à l'issue de la phase 1), par absorption réfrigérée dans un solvant tel que le toluène,

3) conversion des hydrocarbures $C_2^+$ provenant de la phase 2) en alkylaromatiques.

La phase 1) de craquage thermique du gaz naturel, plus particulièrement du méthane, s'effectue en présence d'hydrogène, à une température élevée comprise généralement entre 900 et 1400°C, par exemple à environ 1200°C, de préférence dans un réacteur multicanaux en carbure de silicium dont les canaux sont parcourus par un fluide caloporteur. Cette réaction, qui génère notamment de l'hydrogène, des hydrocarbures $C_2^+$ (en particulier de l'éthylène et de l'acétylène), des hydrocarbures lourds (hydrocarbures $C_7^+$ (sauf toluène)), est par exemple décrite dans les demandes de brevet FR-A-2589859, 2596046 et 2600329.

La phase 2) de séparation des hydrocarbures $C_2^+$ comprend avantageusement les étapes successives suivantes :

a) envoi de l'effluent issu de la phase 1) dans une colonne de quench où lesdits hydrocarbures lourds sont au moins en partie absorbés dans un solvant lourd, du type gazole ou huile, et soutirés en fond de colonne de quench, et où une phase gazeuse est recueillie en tête de colonne de quench,

b) compression de la phase gazeuse issue de l'étape a), afin d'éliminer au moins en majeure partie les produits lourds restant (hydrocarbures $C_7^+$ (sauf toluène) et solvant lourd)) de ladite phase gazeuse, habituellement entre 1 et 7 MPa, de préférence entre 3 et 5 MPa, par exemple à 4 MPa environ,

c) séparation de l'hydrogène excédentaire, généré par la phase 1) et contenu dans la phase gazeuse issue de l'étape b), par perméation gazeuse,

d) absorption réfrigérée des hydrocarbures $C_2^+$, contenus dans la phase gazeuse issue de l'étape c), dans un solvant tel que le toluène de manière à séparer sensiblement, d'une part, lesdits hydrocarbures $C_2^+$ (en particulier l'éthylène et l'acétylène), en phase liquide, et, d'autre part, l'hydrogène et le méthane, en phase gazeuse.

La phase 3) de conversion des hydrocarbures $C_2^+$ en alkylaromatiques comprend avantageusement les étapes successives suivantes :

i) hydrogénation sélective de la phase liquide renfermant les hydrocarbures $C_2^+$ (en particulier l'éthylène et l'acétylène) de manière à transformer sélectivement l'acétylène en éthylène,

ii) élimination au moins partielle du toluène contenu dans l'effluent issu de l'étape i), ledit toluène étant généralement recyclé vers l'étape d),

iii) alkylation du toluène (pouvant être différent de celui éliminé dans l'étape ii)), par des hydrocarbures $C_2^+$ (en particulier l'éthylène) contenus dans le mélange issu de l'étape ii).

L'acétylène étant un poison des catalyseurs d'alkylation du toluène par l'éthylène, il est indispensable de le transformer au préalable par hydrogénation sélective (étape i)). Cette réaction d'hydrogénation sélective de l'acétylène est effectuée habituellement en présence d'au moins un catalyseur disposé par exemple sous forme d'un lit fixe, ledit catalyseur étant de préférence formé par au moins un métal hydrogénant, le plus souvent du palladium, déposé sur un support sensiblement neutre ; un tel catalyseur est notamment décrit dans la demande de brevet français FR-A-2552078. La température de réaction (étape i)) est généralement comprise entre environ 0 et 100°C, sous une pression d'environ 0,1 à 10 MPa (de préférence, la température est d'environ 10 à 60°C et la pression est d'environ 1 à 6 MPa), avec un débit d'hydrocarbures liquides (vitesse spatiale) d'environ 2 à 20 volumes par volume de catalyseur et par heure et avec un rapport molaire hydrogène/acétylène à l'entrée du réacteur d'hydrogénation compris entre 0,5 et 5.

La réaction d'alkylation du toluène notamment par l'éthylène (étape iii)) est effectuée généralement en présence d'au moins un catalyseur zéolithique acide disposé par exemple sous forme d'un lit fixe, habituellement à une température comprise entre environ 50 et 350°C (de préférence entre environ 150 et 300°C), sous une pression de 1 à 10 MPa (de préférence entre 2 et 7 MPa), avec un débit d'hydrocarbures liquides (vitesse spatiale) d'environ 0,2 à 5 volumes par volume de catalyseur et par heure et avec un rapport molaire toluène/éthylène à l'entrée du réacteur d'alkylation compris entre 0,5 et 20 (de préférence entre 2 et 15). Le catalyseur utilisé dans l'étape iii) est de préférence à base d'une mordénite désaluminée de rapport atomique Si/Al global compris entre 20 et 60 ; ce catalyseur est notamment décrit dans les demandes de brevet français n° 87/12932, 88/14099 et 88/17164.

Un mode particulier de réalisation de l'invention est décrit ci-dessous :

- Du gaz naturel comprenant essentiellement du méthane est soumis à une réaction de craquage thermique à haute température dans un réacteur multicanaux en carbure de silicium ; à la sortie dudit réacteur, l'effluent est constitué d'hydrogène, de méthane, d'acétylène, d'éthylène, d'éthane, de propène, de butanes, de benzène, de toluène et d'hydrocarbures $C_7$.

- L'effluent en sortie du réacteur de craquage thermique est refroidi vers 200-350°C et injecté dans une colonne de quench dans laquelle les hydrocarbures les plus lourds ainsi que les suies sont absorbés dans un solvant lourd du type gazole ou huile. La phase liquide formée est pompée en fond de la colonne de quench, refroidie et recyclée en tête ; un appoint de solvant pur et un soutirage sont en général effectués de manière à éviter une accumulation de produit absorbé.

- La phase gazeuse issue de la colonne de quench est comprimée entre 3 et 5 MPa, par exemple à 4 MPa. Compte tenu du taux de compression, cette dernière est de préférence effectuée en plusieurs étages avec refroidissement intermédiaire. En sortie de compresseur, le flux est ramené à une température voisine de l'ambiante (15 à 25°C), afin de condenser les traces de composés lourds (notamment benzène et hydrocarbures $C_7$), qui sont habituellement recyclés vers la colonne de quench.

- L'excédent d'hydrogène généré par le craquage du méthane est séparé par perméation gazeuse. Pour ce faire, le flux est préchauffé pour s'éloigner du point de rosée, puis introduit dans un perméateur. L'hydrogène extrait est à faible pression et pratiquement pur. Ce flux d'hydrogène peut éventuellement être recyclé à l'étape ultérieure d'hydrogénation sélective de l'acétylène.

- La fraction d'hydrocarbures $C_2^+$ est ensuite séparée de l'hydrogène et du méthane non transformé (recyclés au réacteur de craquage thermique) par absorption. Cette opération se fait par exemple par contact à contrecourant avec du toluène froid. La nécessité d'avoir un taux de récupération élevé des $C_2^+$ (généralement supérieur à 97 %) impose le plus souvent des conditions opératoires de forte pression (par exemple environ 4MPa), de basse température (par exemple environ - 40°C) et un taux de solvant habituellement supérieur à 1. La phase liquide issue de l'étape d'absorption réfrigérée, qui contient également une proportion de méthane coabsorbé dans le solvant, est envoyée en totalité à l'étape d'hydrogénation sélective de l'acétylène.

- La phase gazeuse sortant en tête de l'unité d'absorption réfrigérée est détendue dans une turbine. Le froid ainsi produit permet d'assurer la totalité de la réfrigération nécessaire à l'absorption. Le travail mécanique de détente est récupéré au niveau des compresseurs. Le flux détendu est en général recyclé au réacteur de craquage thermique.

- La phase liquide issue de l'étape d'absorption réfrigérée subit ensuite une hydrogénation sélective ayant pour but de transformer sélectivement l'acétylène en éthylène. L'opération est généralement effectuée avec un excès d'hydrogène (par exemple de 10 à 35 %) par rapport à la stoechiométrie. L'hydrogène peut provenir soit du perméateur (ce qui entraîne une compression supplémentaire), soit du mélange d'hydrogène et de méthane de l'étape d'absorption réfrigérée.

- La quantité de toluène contenue dans le flux issu de l'étape d'hydrogénation sélective est généralement excédentaire pour l'étape d'alkylation. En effet, le rapport molaire toluène/éthylène est en général supérieur à 20, alors qu'une valeur comprise entre 7 et 15 est de préférence requise pour la réaction d'alkylation. Par ailleurs, une séparation par distillation du toluène et des alkyltoluènes prévue après l'alkylation serait coûteuse, puisque le constituant majoritaire, à savoir le toluène, devrait être soutiré en tête de colonne. Il est donc nécessaire de séparer avant l'alkylation le toluène non indispensable à cette étape. Pour cette raison, à l'issue de l'étape d'hydrogénation sélective, le toluène est au moins partiellement éliminé : le mélange est détendu à basse pression et fractionné dans une colonne ; le toluène soutiré en fond est en général recyclé vers l'étape d'absorption réfrigérée, le gaz de tête étant recomprimé et envoyé à l'étape d'alkylation.

- En sortie de compresseur, ce gaz de tête contenant l'éthylène est mélangé à du toluène en excès (généralement 7 à 15 fois la stoechiométrie). Le mélange obtenu est chauffé entre environ 240 et 300°C, puis envoyé dans un réacteur d'alkylation où l'éthylène et au moins une partie du toluène sont transformés notamment en éthyltoluène et en polyéthyltoluènes.

- A la sortie du réacteur d'alkylation, l'effluent comprenant les alkyltoluènes et le toluène en excès est détendu et injecté dans une seconde colonne à distiller. Le flux sortant en tête de colonne est essentiellement composé d'hydrogène, de méthane, d'éthane, de butanes et de toluène. Ce flux est refroidi, puis envoyé dans un condenseur. La fraction gazeuse résiduaire (essentiellement du méthane et de l'hydrogène) sortant en tête du condenseur est généralement recyclée au réacteur de craquage thermique. La fraction liquide soutirée en fond du condenseur (essentiellement du toluène) est en partie injectée

3

en reflux dans la seconde colonne à distiller et en partie recyclée en amont du réacteur d'alkylation avec, en général, un appoint de toluène pour compenser la part transformée en alkyltoluènes. Les alkyltoluènes sont récupérés en fond de la seconde colonne à distiller et par exemple envoyés au pool essence d'une raffinerie.

L'exemple suivant illustre l'invention sans toutefois en limiter la portée.

EXEMPLE

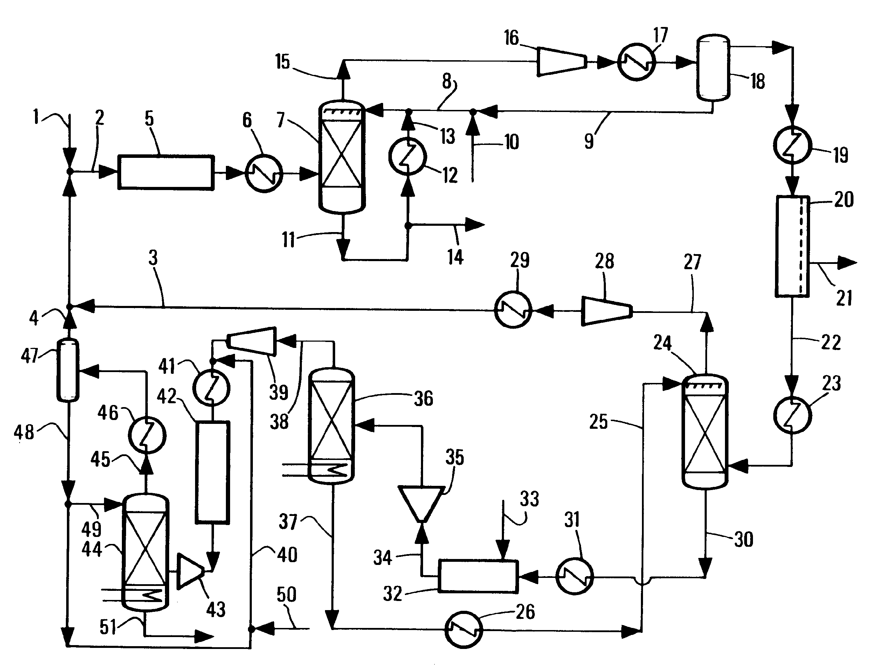

Il est illustré par la figure unique.

Une charge (2) constituée par un mélange de gaz naturel frais (1), comprenant essentiellement du méthane, de gaz (3) en provenance de la tête de l'absorbeur (24) et de gaz en provenance de la tête (4) du condenseur (47) est introduit dans une section de craquage thermique (5).

Ladite charge (2) a la composition suivante (exprimée en kilogramme) :

| | | |
|---|---|---:|
| − hydrogène | : | 661 |
| − méthane | : | 4988 |
| − éthylène | : | 3 |
| − éthane | : | 35 |
| − n-butane | : | 34 |
| − toluène | : | 2 |
| | | 5723 |

La charge est ainsi préchauffée vers 600°C puis craquée dans une zone de pyrolyse multicanaux en carbure de silicium. Un fluide caloporteur formé de fumées de combustion de brûleur à 1400°C environ est envoyé dans les canaux destinés à cet usage à un débit tel que la température du mélange effluent en sortie de pyrolyse soit d'environ 1200°C ; le temps de séjour de la charge dans cette zone est de 300 millisecondes environ. Après passage dans une zone de trempe (alimentée par de l'air comme fluide réfrigérant), l'effluent gazeux est à une température d'environ 250°C.

Cet effluent gazeux sortant de (5) a, après refroidissement (6) à la température ambiante, la composition suivante (exprimée en kilogramme) :

| | | |
|---|---|---:|
| − hydrogène | : | 948 |
| − méthane | : | 3680 |
| − acétylène | : | 424 |
| − éthylène | : | 339 |
| − éthane | : | 16 |
| − propène | : | 9 |
| − n-butane | : | 34 |
| − benzène | : | 190 |
| − toluène | : | 6 |
| − hydrocarbures $C_7$ | : | 77 |
| | | 5723 |

Ceci correspond à un taux de transformation par passe du méthane de 26,2 % environ.

Ces 5723 kg d'effluent gazeux sont ensuite envoyés dans une colonne de quench (7), en même temps que 1112 kg d'un solvant lourd comprenant du solvant recyclé (9) et du solvant frais d'appoint (10). La composition de ces 1112 kg est la suivante :

```
- hydrogène            :      0,02
- méthane              :      1,12
- acétylène            :      0,52
- éthylène             :      0,28
- n-butane             :      0,58
- benzène              :     30,42
- toluène              :      2,76
- hydrocarbures C7     :     32,86
- gazole               :   1043,80
                            1112
```

En fond de la colonne de quench, on soutire 1056 kg de produits lourds qui sont refroidis (12) et recyclés (13) en haut de la colonne de quench, après un appoint éventuel de solvant lourd (10). Ces 1056 kg de produits lourds ont la composition suivante :

```
- méthane              :      0,16
- benzène              :      4,68
- toluène              :      0,92
- hydrocarbures C7     :     67,84
- gazole               :    982,40
                            1056
```

Un soutirage (14) peut éventuellement être effectué afin d'éviter notamment une accumulation de produits absorbés.

En tête (15) de cette colonne de quench, on recueille 5779 kg de phase gazeuse de composition suivante :

```
- hydrogène            :    948,02
- méthane              :   3680,96
- acétylène            :    424,52
- éthylène             :    339,28
- éthane               :     16
- propène              :      9
- n- butane            :     34,58
- benzène              :    215,74
- toluène              :      7,84
- hydrocarbures C7     :     42,02
- solvant lourd        :     61,04
                            5779
```

Cette phase gazeuse est ensuite comprimée à 4 MPa (16) puis ramenée à la température ambiante (17) ; de cette manière on condense 130 kg de produits lourds que l'on sépare (18) puis recycle (9) vers la colonne de quench (7). Ces 130 kg de produits lourds ont la composition suivante :

|  |  |  |
|---|---|---|
| - hydrogène | : | 0,02 |
| - méthane | : | 0,96 |
| - acétylène | : | 0,52 |
| - éthylène | : | 0,28 |
| - n-butane | : | 0,58 |
| - benzène | : | 30,42 |
| - toluène | : | 2,76 |
| - hydrocarbures $C_7$ | : | 33,42 |
| - solvant lourd | : | 61,04 |
|  |  | 130 |

Après séparation de ces produits lourds recyclés au quench, 5649 kg de gaz sont préchauffés (19) pour s'éloigner du point de rosée, et introduits dans un perméateur (20) de manière à diminuer l'excès d'hydrogène généré par le craquage thermique du méthane. Ces 5649 kg de gaz ont la composition suivante :

|  |  |  |
|---|---|---|
| - hydrogène | : | 948 |
| - méthane | : | 3680 |
| - acétylène | : | 424 |
| - éthylène | : | 339 |
| - éthane | : | 16 |
| - propène | : | 9 |
| - n-butane | : | 34 |
| - benzène | : | 185,32 |
| - toluène | : | 5,08 |
| - hydrocarbures $C_7$ | : | 8,60 |
|  |  | 5649 |

A la sortie du perméateur, on recueille, d'une part, 288 kg d'hydrogène pur (21) qui peut être utilisé dans une autre section de la raffinerie (par exemple en partie dans l'étape ultérieure d'hydrogénation sélective de l'acétylène), et, d'autre part, 5361 kg d'effluent gazeux (22) qui est, après réfrigération (23), envoyé vers un absorbeur (24). Cet effluent a la composition suivante (exprimée en kilogramme) :

```
- hydrogène          :   660
- méthane            :  3680
- acétylène          :   424
- éthylène           :   339
- éthane             :    16
- propène            :     9
- n-butane           :    34
- benzène            :  185,32
- toluène            :    5,08
- hydrocarbures C7   :    8,60
                         5361
```

Dans l'absorbeur (24), la fraction d'hydrocarbures $C_2^+$ est sensiblement séparée de l'hydrogène et du méthane, par contact à contre-courant avec 68120 kg de toluène (25) refroidi (26) à environ - 40°C en provenance du fond (37) de la colonne de distillation (36).

Après réaction, on recueille en tête (27) de l'absorbeur 3158 kg de gaz qui sont détendus (28), réchauffés (29) et recyclés (3) vers la section de craquage thermique (5). Ces 3158 kg de gaz ont la composition suivante :

```
- hydrogène          :   655
- méthane            :  2498
- éthylène           :     3
- toluène            :     2
                         3158
```

En fond (30) de l'absorbeur, on soutire 70323 kg de phase liquide de composition suivante :

```
- hydrogène          :     5
- méthane            :  1182
- acétylène          :   424
- éthylène           :   336
- éthane             :    16
- propène            :     9
- n-butane           :    34
- benzène            :  185,4
- toluène            : 68123
- hydrocarbures C7   :    8,6
                        70323
```

Cette phase liquide est ensuite réchauffée (31), puis envoyée vers la section d'hydrogénation sélective (32), où en présence d'un catalyseur à base de palladium, l'acétylène est hydrogéné dans les conditions opératoires suivantes :

- température : 20°C
- pression : 4 MPa
- débit horaire de charge liquide égal à 10 fois le volume du catalyseur
- rapport molaire hydrogène/acétylène = 1,2, c'est-à-dire que 34 kg d'hydrogène sont ajoutés (33).

A la sortie (34) de la section d'hydrogénation sélective, on recueille 70357 kg d'effluent de composition suivante :

|  |  |  |
|---|---|---|
| - hydrogène | : | 6 |
| - méthane | : | 1182 |
| - acétylène | : | 17 |
| - éthylène | : | 757 |
| - éthane | : | 35 |
| - propène | : | 9 |
| - n-butane | : | 34 |
| - benzène | : | 185,4 |
| - toluène | : | 68123 |
| - hydrocarbures $C_7$ | : | 8,6 |
|  |  | 70357 |

Cet effluent est ensuite détendu (35), puis envoyé dans une première colonne à distiller (36) pour séparer le toluène des hydrocarbures plus légers.

En fond (36) de cette colonne à distiller, on soutire 68120 kg de toluène qui sont recyclés en tête de l'absorbeur (24).

En tête (38) de cette colonne à distiller, on recueille 2237 kg de phase gazeuse de composition suivante :

|  |  |  |
|---|---|---|
| - hydrogène | : | 6 |
| - méthane | : | 1182 |
| - acétylène | : | 17 |
| - éthylène | : | 757 |
| - éthane | : | 35 |
| - propène | : | 9 |
| - n-butane | : | 34 |
| - benzène | : | 185,4 |
| - toluène | : | 3 |
| - hydrocarbures $C_7$ | : | 8,6 |
|  |  | 2237 |

Cette phase gazeuse est recomprimée (39) et mélangée avec 25538 kg de toluène (40) ; le mélange résultant, après préchauffage (41), est envoyé dans un réacteur d'alkylation (42) où les oléfines sont transformées en hydrocarbures alkylaromatiques, en présence d'une mordénite désaluminée de rapport atomique Si/Al global d'environ 35, dans les conditions opératoires suivantes :
- température : 270°C

- pression : 4 MPa
- débit horaire de charge liquide égal à 2 fois le volume du catalyseur.

A la sortie du réacteur d'alkylation, on recueille 27775 kg de produit de composition suivante :

| | | |
|---|---|---:|
| - hydrogène | : | 6 |
| - méthane | : | 1182 |
| - éthane | : | 35 |
| - n-butane | : | 34 |
| - benzène | : | 167 |
| - toluène | : | 22917 |
| - hydrocarbures $C_7$ | : | 9 |
| - éthylbenzène | : | 25 |
| - méthyléthylbenzènes | : | 3031 |
| - méthyldiéthylbenzènes | : | 329 |
| - méthylisopropylbenzènes | : | 40 |
| | | 27775 |

Après détente (43), ce produit est envoyé dans une seconde colonne à distiller (44).

Le produit sortant en tête de cette colonne à distiller est refroidi (46), puis envoyé dans un condenseur (47).

En tête (4) de ce condenseur, on recueille 1257 kg de gaz de composition suivante :

| | | |
|---|---|---:|
| - hydrogène | : | 6 |
| - méthane | : | 1182 |
| - éthane | : | 35 |
| - n-butane | : | 34 |
| | | 1257 |

Ce gaz est ensuite recyclé vers la section de craquage thermique (5).

En fond (48) de ce condenseur, on recueille 23084 kg de phase liquide qui est recyclée, en partie, vers le sommet (49) de la seconde colonne à distiller (44) pour servir de reflux et, en partie, en amont (40) du réacteur d'alkylation, après ajout d'un appoint de toluène (50).

En fond (51) de la seconde colonne à distiller (44), on recueille 3434 kg de produit de composition suivante :

| | | |
|---|---|---:|
| - hydrocarbures $C_7$ | : | 9 |
| - éthylbenzène | : | 25 |
| - méthyléthylbenzènes | : | 3031 |
| - méthyldiéthylbenzènes | : | 329 |
| - méthylisopropylbenzènes | : | 40 |
| | | 3434 |

Ces 3434 kg constituent une bonne base pour supercarburant et peuvent donc être envoyés au pool essence de la raffinerie.

- distillation ASTM :
  - point initial : 99°C

9

- point final      : 212°C
- indices d'octanes :
  - RON clair      : 110
  - MON clair      : 101

## Revendications

1. Procédé de production d'hydrocarbures alkylaromatiques à partir de gaz naturel renfermant du méthane caractérisé en ce qu'il comprend les trois phases successives suivantes :

   1) craquage thermique du gaz naturel avec formation d'hydrogène et d'hydrocarbures $C_2^+$, en particulier d'éthylène et d'acétylène,

   2) séparation des hydrocarbures $C_2^+$, en particulier de l'éthylène et de l'acétylène, obtenus à l'issue de la phase 1), par absorption réfrigérée dans un solvant,

   3) conversion des hydrocarbures $C_2^+$ provenant de la phase 2) en alkylaromatiques.

2. Procédé selon la revendication 1 caractérisé en ce que la phase 1) s'effectue en présence d'hydrogène, à une température comprise entre 900 et 1400°C, dans un réacteur multicanaux en carbure de silicium dont les canaux sont parcourus par un fluide caloporteur.

3. Procédé selon l'une des revendications 1 et 2 caractérisé en ce que la phase 2) comprend les étapes successives suivantes :

   a) envoi de l'effluent issu de la phase 1) dans une colonne de quench où les hydrocarbures lourds obtenus à la phase 1) sont au moins en partie absorbés dans un solvant lourd et soutirés en fond de ladite colonne de quench, et où une phase gazeuse est recueillie en tête de ladite colonne de quench,

   b) compression entre 1 et 7 MPa de la phase gazeuse issue de l'étape a), afin d'éliminer au moins en partie les produits lourds restant de ladite phase gazeuse,

   c) séparation de l'hydrogène excédentaire, généré par la phase 1) et contenu dans la phase gazeuse issue de l'étape b), par perméation gazeuse,

   d) absorption refrigérée des hydrocarbures $C_2^+$, en particulier de l'éthylène et de l'acétylène, contenus dans la phase gazeuse issue de l'étape c), dans du toluène, de manière à séparer sensiblement, d'une part, lesdits hydrocarbures $C_2^+$, en particulier l'éthylène et l'acétylène, et, d'autre part, l'hydrogène et le méthane.

4. Procédé selon la revendication 3 caractérisé en ce que l'hydrogène et le méthane séparés dans l'étape d) sont recyclés vers la phase 1).

5. Procédé selon l'une des revendications 1 à 4 caractérisé en ce que la phase 3) comprend les étapes successives suivantes :

   i) hydrogénation sélective de la phase renfermant les hydrocarbures $C_2^+$, en particulier l'éthylène et l'acétylène, issue de la phase 2), de manière à transformer sélectivement l'acétylène en éthylène,

   ii) élimination au moins partielle du toluène contenu dans l'effluent issu de l'étape i),

   iii) alkylation du toluène par des hydrocarbures $C_2^+$, en particulier l'éthylène, contenus dans le mélange issu de l'étape ii).

6. Procédé selon les revendications 3 et 5 caractérisé en ce que l'hydrogène excédentaire séparé par perméation gazeuse dans l'étape c) est recyclé vers l'étape i).

7. Procédé selon l'une des revendications 5 et 6 caractérisé en ce que l'étape i) s'effectue en présence d'au moins un catalyseur formé par au moins un métal hydrogénant.

8. Procédé selon l'une des revendications 5 à 7 caractérisé en ce que l'étape iii) s'effectue en présence d'au moins un catalyseur à base d'une mordénite désaluminée de rapport atomique Si/Al global compris entre 20 et 60.

## Patentansprüche

1. Verfahren zur Herstellung von alkylaromatischen Kohlenwasserstoffen aus Erdgas, welches Methan ent-

**10**

EP 0 431 990 B1

hält, dadurch gekennzeichnet, daß es die folgenden aufein-anderfolgenden Schritte umfaßt:

1) thermisches Cracken von Erdgas unter Bildung von Wasserstoff und $C_2^+$-Kohlenwasserstoff, insbesondere von Ethylen und Acetylen,

2) Abtrennung der aus Stufe 1) erhaltenen $C_2^+$-Kohlenwasserstoffe, insbesondere von Ethylen und von Acetylen, durch Gefrierabsorption in einem Lösungsmittel,

3) Umwandlung der aus Stufe 2) stammenden $C_2^+$-Kohlenwasserstoffe in Alkylaromaten.

2. Verfahren gemäß Anspruch 1, dadurch gekennzeichnet, daß die Stufe 2) in Gegenwart von Wasserstoff bei einer Temperatur, enthalten zwischen 900 und 1400°C, in einem mehrwegigen Siliciumcarbid-Reaktor, dessen Kanäle von einem flüssigen Kühlmittel durchzogen sind, durchgeführt wird.

3. Verfahren gemäß einem der Ansprüche 1 und 2, dadurch gekennzeichnet, daß die Stufe 2) die folgenden aufeinanderfolgenden Schritte umfaßt:

a) Einleitung der aus Stufe 1) stammenden Abflüsse in eine Quench-Kolonne, wo die in Stufe 1) erhaltenen Kohlenwasserstoffe wenigstens teilweise in einem schweren Lösungsmittel absorbiert und am Boden der Quench-Kolonne abgezogen werden, und wo eine Gasphase am Kopf der Quench-Kolonne gesammelt wird,

b) Verdichtung der aus Schritt a) stammenden Gasphase auf zwischen 1 und 7 MPa, um wenigstens teilweise die schweren Produkte, welche in der Gasphase verblieben sind, zu entfernen,

c) Abtrennung des überschüssigen in Stufe 1) erzeugten und in der aus Schritt b) stammenden Gasphase enthaltenen Wasserstoffs durch Gaspermeation,

d) Gefrierabsorption der $C_2^+$-Kohlenwasserstoffe, insbesondere von Ethylen und von Acetylen, welche in der aus Schritt c) stammenden Gasphase enthalten sind, in Toluol, so daß zum einen Teil die $C_2^+$-Kohlenwasserstoffe, insbesondere Ethylen und Acetylen, und zum anderen Teil Wasserstoff und Methan genau abgetrennt werden.

4. Verfahren gemäß Anspruch 3, dadurch gekennzeichnet, daß in Schritt d) abgetrennter Wasserstoff und abgetrenntes Methan in die Stufe 1) zurückgeführt werden.

5. Verfahren gemäß einem der Ansprüche 1 bis 4, dadurch gekennzeichnet, daß die Stufe 3) die folgenden aufeinanderfolgenden Schritte umfaßt:

i) selektive Hydrierung der aus Stufe 2) stammenden, die $C_2^+$-Kohlenwasserstoffe, insbesondere Ethylen und Acetylen enthaltenden Phase, so daß Acetylen selektiv in Ethylen umgewandelt wird,

ii) wenigstens teilweises Entfernen des Toluols, welches in dem aus Schritt i) stammenden Abfluß enthalten ist,

iii) Alkylierung von Toluol mit den $C_2^+$-Kohlenwasserstoffen, insbesondere Ethylen, welche in der aus Schritt ii) stammenden Mischung enthalten sind.

6. Verfahren gemäß der Ansprüche 3 und 5, dadurch gekennzeichnet, daß der in Schritt c) durch Gaspermeation abgetrennte überschüssige Wasserstoff in Schritt 1) zurückgeführt wird.

7. Verfahren gemäß einem der Ansprüche 5 und 6, dadurch gekennzeichnet, daß der Schritt i) in Gegenwart wenigstens eines aus wenigstens einem hydrierenden Metall gebildeten Katalysators bewirkt wird.

8. Verfahren gemäß einem der Ansprüche 5 bis 7, dadurch gekennzeichnet, daß der Schritt iii) in Gegenwart wenigstens eines Katalysators auf der Basis eines entaluminisierten Mordenits mit einem Gesamtatomverhältnis Si/Al, enthalten zwischen 20 und 60, bewirkt wird.

**Claims**

1. A process for producing alkyraromatic hydrocarbons from natural gas containing methane, characterized in that it comprises the following three successive stages :

1) thermal cracking of the natural gas with forming of hydrogen and $C_2^+$ hydrocarbons, particularly ethylene and acetylene,

2) separation of the $C_2^+$ hydrocarbons, particularly of the ethylene and the acetylene, obtained at the end of stage 1), by cooled absorption in a solvent,

3) conversion of the $C_2^+$ hydrocarbons from stage 2) into alkylaromatics.

11

2. A process according to claim 1, characterized in that stage 1) is carried out in the presence of hydrogen, at a temperature ranging from 900 to 1,400°C, in a multichannel reactor made of silicon carbide through whose channels a heat-carrying fluid flows.

3. A process according to any one of claims 1 and 2, characterized in that stage 2) comprises the following successive stages :

a) sending of the effluent from stage 1) into a quenching tower where the heavy hydrocarbons obtained in stage 1) are at least partly absorbed in a heavy solvent and withdrawn at the bottom of said quenching tower, and where a gaseous phase is collected at the head of said quenching tower,

b) compression between 1 and 7 MPa of the gaseous phase from stage a), in order to remove at least partly the remaining heavy products from said gaseous phase,

c) separation of the excess hydrogen, generated by stage 1) and contained in the gaseous phase from stage b), by gaseous permeation,

d) cooled absorption of the $C_2^+$ hydrocarbons, particularly ethylene and acetylene, contained in the gaseous phase from stage c), in toluene, in order to substantially separate, on one hand, said $C_2^+$ hydrocarbons, particularly ethylene and acetylene, and, on the other hand, the hydrogen and the methane.

4. A process according to claim 3, characterized in that the hydrogen and the methane separated in stage d) are recycled to stage 1).

5. A process according to any one of claims 1 to 4, characterized in that stage 3) comprises the following successive stages :

i) selective hydrogenation of the phase comprising the $C_2^+$ hydrocarbons, particularly ethylene and acetylene, from stage 2), in order to selectively convert the acetylene into ethylene,

ii) at least partial removal of the toluene contained in the effluent from stage i),

iii) alkylation of the toluene by $C_2^+$ hydrocarbons, particularly ethylene, contained in the mixture from stage ii).

6. A process according to claims 3 and 5, characterized in that the excess hydrogen separated by gaseous permeation in stage c) is recycled to stage i).

7. A process according to any one of claims 5 and 6, characterized in that stage i) is performed in the presence of at least one catalyst formed by at least one hydrogenizing metal.

8. A process according to any one of claims 5 to 7, characterized in that stage iii) is performed in the presence of at least one catalyst based on a dealuminized mordenite with a total atomic ratio Si/Al ranging from 20 to 60.